# EUROPÄISCHE PATENTSCHRIFT

(11) **EP 2 547 648 B1**
(45) Veröffentlichungstag und Bekanntmachung des Hinweises auf die Patenterteilung: **10.07.2013**
(21) Anmeldenummer: 11710452.1
(22) Anmeldetag: 15.03.2011
(51) Int. Cl.: C07C 227/18, C07C 229/16, C07C 229/24

(54) **VERFAHREN ZUR HERSTELLUNG NEBENPRODUKTARMER AMINOCARBOXYLATE**
METHOD FOR PRODUCING AMINOCARBOXYLATES LOW IN BY-PRODUCT
PROCÉDÉ DE PRODUCTION D'AMINOCARBOXYLATES À FAIBLE TENEUR EN SOUS-PRODUITS

(30) Priorität: 18.03.2010 EP 10156963
(43) Veröffentlichungstag der Anmeldung: 23.01.2013
(73) Patentinhaber: BASF SE, 67056 Ludwigshafen (DE)
(72) Erfinder: BAUMANN, Robert, 68529 Mannheim (DE); BIEL, Markus Christian, 68159 Mannheim (DE); BRASCHE, Gordon, 60594 Frankfurt (DE); FRANZKE, Axel, 68161 Mannheim (DE); HEIDENFELDER, Thomas, Randolph NJ 07869 (US); KLINGELHOEFER, Paul, 68165 Mannheim (DE); OFTRING, Alfred, 67098 Bad Dürkheim (DE); SCHRÖTER, Marie Katrin, 67125 Dannstadt-Schauernheim (DE)
(86) Internationale Anmeldenummer: PCT/EP2011/053869
(87) Internationale Veröffentlichungsnummer: WO 2011/113822

(56) Entgegenhaltungen:
- WO-A1-00/32310
- DE-B- 1 020 347
- US-A- 3 907 745

## Beschreibung

Die vorliegende Erfindung betrifft ein Verfahren zur Herstellung von Aminocarboxylaten, ausgehend von den Aminen 1 bzw. 4, durch Anwendung einer Reaktionssequenz aus Ethoxylierung zu den Aminoalkoholen 2 bzw. 5 und nachfolgender oxidativer Dehydrierung zu den entsprechenden Aminocarboxylaten 3 bzw. 6 (zum Beispiel den Alkali- bzw. Erdalkalisalzen der Komplexbildner MGDA (Methylglycindiessigsäure), EDTA (Ethylendiamintetraessigsäure) oder GLDA (Glutaminsäurediessigsäure) bzw. deren freien Säuren).

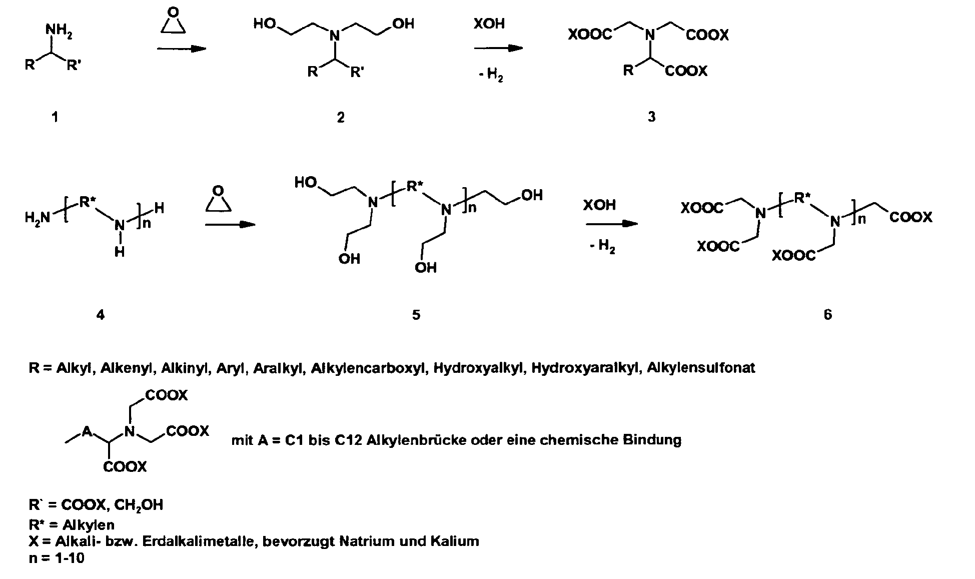

Die Ethoxylierung von Aminen wird im industriellen Maßstab typischerweise bei Temperaturen größer 120 °C durchgeführt. So erfolgt die Herstellung von Ethanolaminen ausgehend von Ammoniak (Lösung von 20 bis 30 Massen-% in Wasser) und Ethylenoxid bei Temperaturen um 150 °C und bei Drücken von 30 bis 150 bar (H.-J. Arpe, Industrielle Organische Chemie). N-Alkylethanolamine werden sogar bei Temperaturen bis 170 °C hergestellt (Ullmann's Enzyklopädie). WO 98/38153 beschreibt die Ethoxylierung von Ethylendiamin in iso-Propanol als Lösungsmittel mit 4 Äquivalenten Ethylenoxid bei Normaldruck und einer Reaktionstemperatur von 140 bis 180 °C. Die entsprechende Ethoxylierung in Reinsubstanz wird in US 3 907 745 bei etwas niedrigeren Temperaturen von 120 bis 130 °C beschrieben.

Die oxidative Dehydrierung von Aminoalkoholen mit Alkalihydroxiden wird üblicherweise unter Druck und bei Temperaturen von 140 bis 220 °C unter Verwendung von Kupfer-Katalysatoren durchgeführt. Die Katalysatoren bestehen z. B aus dotiertem oder undotiertem Raney-Cu (z. B. in EP 1 125 633, EP 1 125 634, WO 04/24091, WO 00/066539, EP 1 067 114, WO 00/032310 beschrieben). Als Dotierstoffe werden in der Regel ein oder mehrere Metalle, wie z. B. Pt, Fe, Cr (EP 1 125 633, EP 1 125 634) Cr, Mo, V, Bi, Sn, Sb, Pb, Ge (WO 04/24091) oder Ag (EP 1 067 114) verwendet.

In anderen Beispielen wird Cu direkt oder über Ankermetalle (z. B. Os, Ir, Rh, Pt, Pd) auf alkalistabile Träger aufgebracht (z. B. WO 01/77054, WO 03/022140, WO 98/50150). Auch Cu-Fällkatalysatoren mit weiteren Metalloxiden wurden beschrieben (z. B. WO 03/051513 (Cu, Fe), EP 0 506 973, WO 98/13140 (Cu, Zr, Ca)). Vereinzelt ist über die Umsetzung an Edelmetall-Systemen berichtet worden (z. B. EP 0 201 957).

Ein Problem bei der Herstellung insbesondere von Komplexbildnern wie MGDA (Methylglycindiessigsäure), EDTA (Ethylendiamintetraessigsäure) oder GLDA (Glutaminsäurediessigsäure) und deren Salzen besteht darin, dass bei einer einfachen Durchführung der beiden Verfahrensschritte relativ hohe Gehalte an Nebenprodukten erhalten werden. Um den Gehalt an derartigen Nebenprodukten im Endprodukt niedrig zu halten, sind daher teure und apparativ aufwändige Reinigungsoperationen des End- und/oder des Zwischenproduktes erforderlich.

Es ist daher Aufgabe der vorliegenden Erfindung ein Verfahren bereitzustellen, das den genannten Nachteil nicht aufweist, das also ein Endprodukt mit niedrigem Nebenproduktanteil liefert und bei dem Reinigungsoperationen des End- und/oder Zwischenproduktes entbehrlich sind.

Diese Aufgabe wurde überraschend gelöst durch das Verfahren gemäß der Ansprüche 1 bis 10:
Erfindungsgemäß wird die Aufgabe gelöst durch ein Verfahren zur Herstellung von Aminocarboxylaten, bei dem in einer ersten Stufe ein Amin bei einer Reaktionstemperatur im Bereich von 30 bis 100 °C zu einem Alkanolamin ethoxyliert wird und das so gebildete Alkanolamin in einer zweiten Stufe oxidativ zu einem Aminocarboxylat dehydriert wird, wobei sich die entstehenden Salze auch in die entsprechenden Aminocarbonsäuren überführen lassen.

Dabei ist ein Verfahren bevorzugt, bei dem das Amin ausgewählt ist aus der Gruppe der Amine der 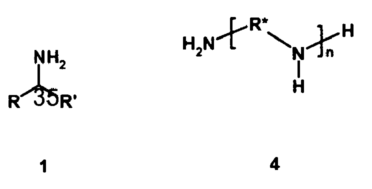 Formel 1 oder 4, wobei
R ein Alkyl-, Alkenyl-, Alkinyl-, Aryl-, Aralkyl-, Alkylencarboxyl-, Hydroxyalkyl-, Hydroxyaralkyl-, Alkylensulfonat oder ein Substituent 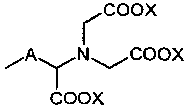 mit A = C1 bis C12 Alkylen-Brücke, oder eine chemische Bindung
R' COOX oder CH₂OH,
R* ein Alkylen-Rest,
X ein Alkali- oder Erdalkalimetall und
n eine Zahl von 1 bis 10 ist.

Besonders bevorzugt handelt es sich bei R um längere Alkyl- bzw. Alkenylreste mit C1 bis C30 Alkyl und C2 bis C30 Alkenyl, Alkylencarboxylate aber auch Alkylensulfonate, Hydroxyalkyl- bzw. Hydroxyarylgruppen und doppelte Alkylglycindiessigsäuren wie Diaminobernsteinsäure (A= "chemische Bindung") oder Diaminopimelinsäure (A = -(CH₂)₃-) mit R= 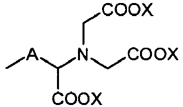 wobei A = eine C1 bis C12 Alkylen-Brücke oder eine chemische Bindung ist

Dabei ist besonders bevorzugt ein Verfahren, bei dem das Amin ausgewählt ist aus der Gruppe bestehend aus Alanin, Glutaminsäure und dessen/deren Salzen sowie Ethylendiamin.

Bezüglich der Verfahrensparameter gibt es bevorzugte Ausführungsformen. So ist ein Verfahren bevorzugt, bei dem die Reaktionstemperatur in der ersten Stufe im Bereich von 40 bis 90 °C, bevorzugt im Bereich von 60 bis 80 °C liegt.

Auch bezüglich des Temperaturverlaufes gibt es bevorzugte Varianten. So ist ein Verfahren bevorzugt, bei dem die Reaktionstemperatur in der ersten Stufe während der Reaktionszeit weniger als 60 °C, vorzugsweise weniger als 40 °C schwankt.

Die Ausführung des Verfahrens als batch-, semi-batch oder konti-Verfahren ist bevorzugt. Ein Verfahren, bei dem (mindestens) ein Reaktor ausgewählt aus der Gruppe bestehend aus Rührkesselreaktor, Schlaufenreaktor und Rohrreaktor verwendet wird, ist besonders bevorzugt. Dies ist unter Verwendung verschiedener Reaktormodelle wie Rührkesselreaktoren verschiedener Bauarten, Schlaufenreaktoren (Gasumlaufreaktor, Tauchstrahlreaktor, Strahldüsenreaktor oder hochbelastete Packungssäule) oder Rohrreaktoren (gasphasenfrei oder mit Gasphase) möglich.

Ein Verfahren, bei dem der Reaktor im Wesentlichen aus einem Material mit einem Wärmeleitfähigkeitskoeffizienten größer 5 W/K*m besteht, ist besonders geeignet. Dabei bedeutet "im Wesentlichen", dass mehr als 50 %, vorzugsweise mehr als 80 % und besonders bevorzugt mehr als 90 % des Reaktormaterials aus einem Material mit entsprechendem Wärmeleitfähigkeitskoeffizienten bestehen.

Als besonders geeignet erweisen sich dafür Materialien bzw. Werkstoffe wie 1.4541 (V2A-Stahl), 1.4571 (V4A-Stahl), 2.4610 (HC4) mit einem Wärmeleitfähigkeitskoeffizienten größer 5 W/K*m, um im technischen Verfahren eine effiziente Wärmeabfuhr zu ermöglichen.

Ebenso ist bevorzugt ein Verfahren, bei dem das Lösungsmittel der ersten Stufe ausgewählt ist aus protischen Lösungsmitteln wie Wasser, Alkoholen, bevorzugt kurzkettigen Alkoholen und insbesondere Methanol, Ethanol, 2-Propanol und/oder polar aprotischen Lösungsmitteln wie Dimethylsulfoxid, Dimethylformamid oder N-Methylpyrrolidon.

Ein Verfahren, bei dem das in der ersten Stufe gebildete Alkanolamin direkt dehydriert wird, stellt eine weitere bevorzugte Ausführungsform dar. Eine direkte Dehydrierung bedeutet, dass solche Verfahren bevorzugt sind, in denen keine auf unterschiedlichen Siedepunkten basierende apparative Abtrennung von Substanzen mit Siedepunkten größer 200 °C (bei Normaldruck) zwischen der ersten und der zweiten Stufe erfolgt. Dies ist apparativ einfacher und spart somit einen Verfahrensschritt bei vergleichbar guter Endproduktqualität.

Besonders bevorzugt ist dabei ein Verfahren, bei dem auch das Endprodukt nicht weiter aufgereinigt wird, sondern direkt in den entsprechenden Anwendungen eingesetzt wird, wie z. B. als Additiv für technische Reinigungsformulierungen für harte Oberflächen aus Metall, Kunststoff, Lack oder Glas, in alkalischen Reinigungsformulierungen für die Getränke- und Nahrungsmittelindustrie, insbesondere für die Flaschenreinigung in der Getränkeindustrie sowie bei der Apparatereinigung in Molkereien, in Brauereien, in der Konserven-, der Backwaren-, der Zucker-, der fettverarbeitenden und der fleischverarbeitenden Industrie, in Geschirrreinigungsformulierungen, insbesondere in phosphatfreien Mitteln für das maschinelle Geschirreinigen in Geschirrspülmaschinen im Haushalt oder in Gewerbebetrieben, z. B. Großküchen oder Restaurants, in Bleichbädern in der Papierindustrie, in photografischen Bleich- und Bleichfixierbädem, bei der Vorbehandlung und Bleichung in der Textilindustrie, in galvanischen Bädem zur Maskierung von verunreinigenden Schwermetallkationen, weiterhin im Bereich der Pflanzenemährung zur Behebung von Schwermetalldefiziten als Kupfer,- Eisen-, Mangan- und Zinkkomplexe. Der Einsatz ist prinzipiell überall dort vorteilhaft, wo technische Verfahren Ausfällungen von Calcium-, Magnesium- oder Schwermetallsalzen stören und verhindert werden sollen (Verhinderung von Ablagerungen und Verkrustungen in Kesseln, Rohrleitungen, Sprühdosen oder allgemein an glatten Oberflächen), außerdem zur Stabilisierung von Phosphaten in alkalischen Entfettungsbädem und Verhinderung der Ausfällung von Kalkseifen, um dadurch das "Anlaufen" von nicht-Eisenoberflächen zu verhindern und die Standzeit von alkalischen Reinigungsbädern zu verlängern. Daneben finden sie Anwendung in Pulver- oder Flüssigwaschmittelformulierungen für die Textilwäsche als Builder und Konservierungsmittel. In Seifen verhindern sie metallkatalysierte oxidative Zersetzungen wie auch in Pharmazeutika, Kosmetika und Nahrungsmitteln.

Die Dehydrierung erfolgt dabei mit Hilfe einer Base aus der Gruppe der Alkali- bzw. Erdalkalihydroxide, bevorzugt NaOH oder KOH, wobei NaOH besonders bevorzugt ist. Die Temperatur der zweiten Stufe liegt üblicherweise im Bereich von 140 bis 240 °C, vorzugsweise im Bereich von 150 bis 210°C und besonders bevorzugt im Bereich von 160 bis 200 °C. Der Druck liegt üblicherweise im Bereich von Normaldruck bis 100 bar, vorzugsweise von 5 bis 50 bar und besonders bevorzugt im Bereich von 8 bis 20 bar und ganz besonders bevorzugt von 10 bis 20 bar.

Ein Verfahren, bei dem die Dehydrierung mit einem Katalysator durchgeführt wird, dessen Haupt- und Nebenbestandteile ausgewählt ist/sind aus den Gruppen 4 bis 12 des Periodensystems, ist dabei besonders bevorzugt, ganz besonders bevorzugt ist ein Verfahren, bei dem die Dehydrierung mit einem Katalysator durchgeführt wird, der (mindestens) ein Metall enthält, das ausgewählt ist aus der Gruppe bestehend aus: Cu, Fe, Co, Ni, Zr, Hf, Ag, Pd und Pt.

Der Katalysator kann z. B. als Pulver oder Formkörper (z. B. Extrudate, Tabletten, ...), als Vollkontakt oder Trägerkatalysator eingesetzt werden und aus Metallen sowie Metalloxiden bestehen.

Ein Verfahren, bei dem der Gehalt an NTA im direkten Produkt der zweiten Stufe kleiner 1 Massen% bezogen auf das Hauptprodukt ist, bildet einen weiteren Gegenstand der vorliegenden Erfindung.

Neben den Salzen (Aminocarboxylate) selbst, sind nach Ansäuem auch die entsprechenden Aminocarbonsäuren zugänglich. Unter dem direkten Produkt der zweiten Stufe versteht man den Reaktionsaustrag, so wie er bei der oxidativen Dehydrierung anfällt. Danach kann im Falle einer Suspensionsfahrweise der Katalysator sedimentiert und abfiltriert werden. Außerdem kann anschließend noch ein gewünschter Wassergehalt eingestellt bzw. eine Bleichung z. B. mit Wasserstoffperoxid oder UV-Licht durchgeführt werden.

Die vorliegende Erfindung wird nachfolgend durch nicht einschränkende Beispiele näher erläutert:

### Beispiel 1:

3,743 kg (20,00 mol) Glutaminsäure Mononatriumsalz Monohydrat wurde in 5,599 kg Wasser suspendiert und mit 1,578 kg (20,00 mol) 50,7 Massen-%iger Natronlauge versetzt. Die resultierende Mischung wurde in einen 20 L-Autoklaven (Material 2.4610) gefüllt und nach entsprechender Inertisierung mit 20 bar Stickstoff beaufschlagt. Anschließend wurde 2,026 kg (46,00 mol) Ethylenoxid bei 40-45 °C innerhalb von 8 h zudosiert und noch weitere 2 h bei dieser Temperatur nachgerührt. Nach der Entfernung der nicht umgesetzten Reste an Ethylenoxid wurde der Autoklav entleert. Auf diese Weise wurde 12,862 kg wässriger Reaktionsaustrag als klare, farblose, viskose Lösung erhalten.

418 g (0,650 mol bezogen auf Glutaminsäure Mononatriumsalz Monohydrat) dieses Rohproduktes wurde mit 53,0 g (1,33 mol) Natriumhydroxidpulver, 12,7 g Wasser und 7,5 g eines gemäß WO 03/051513 hergestellten Kupfer-Eisenkatalysators in einem 1,2 L-Autoklaven (Material 2.4610) vorgelegt. Der Reaktor wurde geschlossen, mit 5 bar Stickstoff beaufschlagt und anschließend auf 190 °C aufgeheizt. Die Temperatur wurde für 6 h gehalten. Die Rührerdrehzahl lag während der ganzen Versuchsdauer bei 700 U/min. Der entstehende Wasserstoff wurde kontinuierlich über ein bei 15 bar regelndes Druckhalteventil abgeführt. Nach Versuchsende wurde der Reaktor bei Raumtemperatur mit Stickstoff gespült und dann entleert. Das Produkt wurde als klare, farblose, viskose Lösung erhalten. Per Eisenbindevermögen wurde ein Gehalt an Glutaminsäure-N,N-diessigsäure Tetranatriumsalz (GLDA-Na₄) von 42,2 Massen-% bestimmt, was einer Ausbeute von 88,6% der Theorie bezogen auf eingesetztes Glutaminsäure Mononatriumsalz Monohydrat entspricht.

### Beispiel 2:

4,365 kg (49,00 mol) Alanin wurde in 2,600 kg Wasser suspendiert und mit 3,920 kg (49,00 mol) 50 Massen-%iger Natronlauge versetzt. Die resultierende Mischung wurde in einen 20 L-Autoklaven (Material 2.4610) gefüllt und nach entsprechender Inertisierung mit 20 bar Stickstoff beaufschlagt. Anschließend wurde 4,749 kg (107,8 mol) Ethylenoxid bei 40-45 °C innerhalb von 8 h zudosiert und noch weitere 2 h bei dieser Temperatur nachgerührt. Nach der Entfernung der nicht umgesetzten Reste an Ethylenoxid wurde der Autoklav entleert. Auf diese Weise wurde 15,597 kg wässriger Reaktionsaustrag als klare, farblose, viskose Lösung erhalten.

328 g (1,03 mol bezogen auf Alanin) dieses Rohproduktes wurde mit 197 g (2,46 mol) 50 Massen-%iger Natronlauge, 18 g Wasser und 45 g Raney-Kupfer (der Firma Evonik Degussa GmbH) in einem 1,7 L-Autoklaven (Material 2.4610) vorgelegt. Der Reaktor wurde geschlossen, mit 5 bar Stickstoff beaufschlagt und anschließend innerhalb von 2,25 h auf 190 °C aufgeheizt. Diese Temperatur wurde für 16 h gehalten. Die Rührerdrehzahl lag während der ganzen Versuchsdauer bei 500 U/min. Der entstehende Wasserstoff wurde kontinuierlich über ein bei 10 bar regelndes Druckhalteventil abgeführt. Nach Versuchsende wurde der Reaktor bei Raumtemperatur mit Stickstoff gespült, der Reaktionsaustrag mit 484 g Wasser verdünnt und dann entleert. Das Produkt wurde als klare, farblose, viskose Lösung erhalten. Mittels HPLC wurde eine Ausbeute an Methylglycin-N,N-diessigsäure Trinatriumsalz (MGDA-Na₃) von 92,0% der Theorie bezogen auf eingesetztes Alanin bestimmt.

### Beispiel 3:

178 g (2,00 mol) Alanin wurde in 106 g Wasser suspendiert und mit 160 g (2,00 mol) 50 Massen-%iger Natronlauge versetzt. Die resultierende Mischung wurde in einen 2,5 L-Autoklaven (Material 1.4571) gefüllt und nach entsprechender Inertisierung mit 1 bar Stickstoff beaufschlagt. Anschließend wurde 189 g (4,30 mol) Ethylenoxid bei 80-89 °C innerhalb von 2 h zudosiert und noch weitere 3 h bei dieser Temperatur nachgerührt. Nach der Entfernung der nicht umgesetzten Reste an Ethylenoxid wurde der Autoklav entleert. Auf diese Weise wurde 624 g wässriger Reaktionsaustrag als klare, farblose, viskose Lösung erhalten.

328 g (1,05 mol bezogen auf Alanin) dieses Rohproduktes wurde mit 208 g (2,60 mol) 50 Massen-%iger Natronlauge, 39 g Wasser und 45 g Raney-Kupfer (der Firma Evonik Degussa GmbH) in einem 1,7 L-Autoklaven (Material 2.4610) vorgelegt. Der Reaktor wurde geschlossen, mit 5 bar Stickstoff beaufschlagt und anschließend innerhalb von 2,25 h auf 190 °C aufgeheizt. Diese Temperatur wurde für 16 h gehalten. Die Rührerdrehzahl lag während der ganzen Versuchsdauer bei 500 U/min. Der entstehende Wasserstoff wurde kontinuierlich über ein bei 10 bar regelndes Druckhalteventil abgeführt. Nach Versuchsende wurde der Reaktor bei Raumtemperatur mit Stickstoff gespült, der Reaktionsaustrag mit 403 g Wasser verdünnt und dann entleert. Das Produkt wurde als klare, farblose, viskose Lösung erhalten. Mittels HPLC wurde eine Ausbeute an Methylglycin-N,N-diessigsäure Trinatriumsalz (MGDA-Na₃) von 91,3% der Theorie bezogen auf eingesetztes Alanin bestimmt.

### Vergleichsbeispiel:

267 g (3,00 mol) Alanin wurde in 159 g Wasser suspendiert und mit 240 g (3,00 mol) 50 Massen-%iger Natronlauge versetzt. Die resultierende Mischung wurde in einen 2,5 L-Autoklaven (Material 1.4571) gefüllt und nach entsprechender Inertisierung mit 20 bar Stickstoff beaufschlagt. Anschließend wurde 291 g (6,60 mol) Ethylenoxid bei 140-145 °C innerhalb von 5 h zudosiert und noch weitere 2 h bei dieser Temperatur nachgerührt. Nach der Entfernung der nicht umgesetzten Reste an Ethylenoxid wurde der Autoklav entleert. Auf diese Weise wurde 930 g wässriger Reaktionsaustrag als klare, gelbliche, viskose Lösung erhalten.

322 g (1,04 mol bezogen auf Alanin) dieses Rohproduktes wurde mit 208 g (2,60 mol) 50 Massen-%iger Natronlauge, 40 g Wasser und 45 g Raney-Kupfer (der Firma Evonik Degussa GmbH) in einem 1,7 L-Autoklaven (Material 2.4610) vorgelegt. Der Reaktor wurde geschlossen, mit 5 bar Stickstoff beaufschlagt und anschließend innerhalb von 2,25 h auf 190 °C aufgeheizt. Diese Temperatur wurde für 16 h gehalten. Die Rührerdrehzahl lag während der ganzen Versuchsdauer bei 500 U/min. Der entstehende Wasserstoff wurde kontinuierlich über ein bei 10 bar regelndes Druckhalteventil abgeführt. Nach Versuchsende wurde der Reaktor bei Raumtemperatur mit Stickstoff gespült, der Reaktionsaustrag mit 424 g Wasser verdünnt und dann entleert. Das Produkt wurde als klare, farblose, viskose Lösung erhalten. Per HPLC wurde trotz Vollumsatz eine Ausbeute an Methylglycin-N,N-diessigsäure Trinatriumsalz (MGDA-Na₃) von lediglich 74,4% der Theorie bezogen auf eingesetztes Alanin bestimmt.

## Patentansprüche

1. Verfahren zur Herstellung von Aminocarboxylaten, bei dem in einer ersten Stufe ein Amin bei einer Reaktionstemperatur im Bereich von 30 bis 100 °C zu einem Alkanolamin ethoxyliert wird und das so gebildete Alkanolamin in einer zweiten Stufe oxidativ zu einem Aminocarboxylat dehydriert wird.

2. Verfahren nach Anspruch 1, bei dem das Amin ausgewählt ist aus der Gruppe bestehend aus Alanin, Glutaminsäure und dessen/deren Salzen sowie Ethylendiamin.

3. Verfahren nach einem der Ansprüche 1 bis 2, bei dem die Reaktionstemperatur in der ersten Stufe während der Reaktionszeit weniger als 60 °C schwankt.

4. Verfahren nach einem der Ansprüche 1 bis 3, das als batch-, semi-batch oder konti-Verfahren durchgeführt wird.

5. Verfahren nach einem der Ansprüche 1 bis 4, bei dem ein Reaktor ausgewählt aus der Gruppe bestehend aus Rührkesselreaktor, Schlaufenreaktor und Rohrreaktor verwendet wird.

6. Verfahren nach einem der Ansprüche 1 bis 5, bei dem der Reaktor im Wesentlichen aus einem Material mit einem Wärmeleitfähigkeitskoeffizienten größer 5 W/K*m besteht.

7. Verfahren nach einem der Ansprüche 1 bis 6, bei dem das Lösungsmittel der ersten Stufe ausgewählt ist aus protischen und/oder polar aprotischen Lösungsmitteln.

8. Verfahren nach einem der Ansprüche 1 bis 7, bei dem das in der ersten Stufe gebildete Alkanolamin direkt dehydriert wird.

9. Verfahren nach einem der Ansprüche 1 bis 8, bei dem die Dehydrierung mit einem Katalysator durchgeführt wird, der ein Metall enthält, das ausgewählt ist aus der Gruppe bestehend aus: Cu, Fe, Co, Ni, Zr, Hf, Ag, Pd und Pt.

10. Verfahren nach einem der Ansprüche 1 bis 9, bei dem der Gehalt an NTA im direkten Produkt der zweiten Stufe kleiner 1 Massen% bezogen auf das Hauptprodukt ist.

## Claims

1. A process for preparing aminocarboxylates, in which, in a first stage, an amine is ethoxylated at a reaction temperature in the range from 30 to 100°C to give an alkanolamine, and the alkanolamine thus formed is dehydrogenated in a second stage oxidatively to give an aminocarboxylate.

2. The process according to claim 1, in which the amine is selected from the group consisting of alanine, glutamic acid and salts thereof, and ethylenediamine.

3. The process according to either of claims 1 and 2, in which the reaction temperature in the first stage varies by less than 60°C over the reaction time.

4. The process according to any of claims 1 to 3, which is performed as a batchwise, semibatchwise or continuous process.

5. The process according to any of claims 1 to 4, in which a reactor selected from the group consisting of stirred tank reactor, loop reactor and tubular reactor is used.

6. The process according to any of claims 1 to 5, in which the reactor consists essentially of a material with a thermal conductivity coefficient greater than 5 W/K*m.

7. The process according to any of claims 1 to 6, in which the solvent of the first stage is selected from protic and/or polar aprotic solvents.

8. The process according to any of claims 1 to 7, in which the alkanolamine formed in the first stage is dehydrogenated directly.

9. The process according to any of claims 1 to 8, in which the dehydrogenation is performed with a catalyst which comprises a metal which is selected from the group consisting of: Cu, Fe, Co, Ni, Zr, Hf, Ag, Pd and Pt.

10. The process according to any of claims 1 to 9, in which the NTA content in the direct product of the second stage is less than 1% by mass, based on the main product.

## Revendications

1. Procédé pour la préparation d'aminocarboxylates, dans lequel, dans une première étape, une amine est éthoxylée en alcanolamine à une température de réaction dans la plage de 30 à 100°C et dans une deuxième étape, l'alcanolamine ainsi formée est déshydrogénée par oxydation en aminocarboxylate.

2. Procédé selon la revendication 1, dans lequel l'amine est choisie dans le groupe constitué par l'alanine, l'acide glutamique et leurs sels ainsi que l'éthylènediamine.

3. Procédé selon l'une quelconque des revendications 1 à 2, dans lequel la température de réaction lors de la première étape, pendant le temps de réaction, fluctue de moins de 60°C.

4. Procédé selon l'une quelconque des revendications 1 à 3, qui est réalisé sous forme de procédé à lots, semi-continu ou continu.

5. Procédé selon l'une quelconque des revendications 1 à 4, dans lequel un réacteur, choisi dans le groupe constitué par un réacteur à cuve agitée, un réacteur à boucle à circulation interne et un réacteur tubulaire, est utilisé.

6. Procédé selon l'une quelconque des revendications 1 à 5, dans lequel le réacteur est essentiellement constitué par un matériau présentant un coefficient de conductibilité thermique supérieur à 5 W/K*m.

7. Procédé selon l'une quelconque des revendications 1 à 6, dans lequel le solvant de la première étape est choisi parmi les solvants protiques et/ou polaires aprotiques.

8. Procédé selon l'une quelconque des revendications 1 à 7, dans lequel l'alcanolamine formée dans la première étape est déshydrogénée directement.

9. Procédé selon l'une quelconque des revendications 1 à 8, dans lequel la déshydrogénation est réalisée avec un catalyseur qui contient un métal choisi dans le groupe constitué par : Cu, Fe, Co, Ni, Zr, Hf, Ag, Pd et Pt.

10. Procédé selon l'une quelconque des revendications 1 à 9, dans lequel la teneur en NTA dans le produit direct de la deuxième étape est inférieure à 1% en masse par rapport au produit principal.
